Europäisches Patentamt

European Patent Office

Office européen des brevets

(11) Publication number: **0 170 608**
**B1**

(12) **EUROPEAN PATENT SPECIFICATION**

(45) Date of publication of patent specification: **20.06.90**

(51) Int. Cl.⁵: **A 61 B 5/117**

(21) Application number: **85630126.2**

(22) Date of filing: **07.08.85**

(54) Spray container for cyanoacrylate esters.

(30) Priority: **07.08.84 US 638379**
**15.10.84 US 661171**
**15.01.85 US 692019**

(43) Date of publication of application:
**05.02.86 Bulletin 86/06**

(45) Publication of the grant of the patent:
**20.06.90 Bulletin 90/25**

(84) Designated Contracting States:
**AT BE CH DE FR GB IT LI LU NL SE**

(56) References cited:
**FR-A-1 565 256**
**US-A-3 523 628**
**US-A-4 297 383**
**US-A-4 407 842**
**US-A-4 477 607**

(73) Proprietor: **PRINT-LOCK CORPORATION**
**8055 West Manchester Avenue Suite 405**
**Playa del Rey California 90293 (US)**

(72) Inventor: **Morton, William P.**
**12260 Missouri Avenue**
**Los Angeles Cal. 90025 (US)**
Inventor: **Butland, Charles L.**
**702 Washington Street Suite 16**
**Marina del Rey Cal. 90292 (US)**

(74) Representative: **Schmitz, Jean-Marie et al**
**OFFICE DENNEMEYER S.à.r.l. P.O. Box 1502**
**L-1015 Luxembourg (LU)**

Courier Press, Leamington Spa, England.

## Description

The present invention concerns a spray container for generating vapors of cyanoacrylate ester comprising a container fitted with a discharge mechanism in which is disposed cyanoacrylate ester dispersed in a hydrocarbon propellant wherein actuating the discharge mechanism generates an atomizate of said cyanoacrylate ester.

The present invention relates to a system for fuming an object suspected of containing latent fingerprints thereon with atomized particles of an alkyl-cyanoacrylate ester and more particularly to a spray container which is filled with a mixture of a solvent and the alkyl-cyanoacrylate ester which the spray container discharges as a fine mist thereby activating an alkyl-cyanoacrylate fuming action in order to generate rapid and prolonged fuming of the suspected object with the atomized particles of the alkyl-cyanoacrylate ester.

The present invention also relates to a system for applying a cyanoacrylate ester adhesive composition onto at least one of two opposing surfaces which are to be chemically joined together and more particularly to a spray container which is filled with a cyanoacrylate ester adhesive composition which the spray container atomizes into an atomizate thereof and discharges as a fine mist onto at least one of the two opposing surfaces.

The FR—A—1 565 256 discloses a spray container for applying adhesive material comprising a cyanoacrylate ester disposed in said container and a discharging means for discharging a fine mist of atomized particles of said cyanoacrylate ester. The cyanoacrylate ester in said spray container is dispersed in a halogenated propellant.

US Patent 4 227 631 entitled Valve for a Spray Container, issued to Hilmar Schneider on October 24, 1980, teaches a valve for an aerosol spray container which has a mixing chamber for mixing a gas propellant with an active ingredient in order to produce a finely atomized spray. The gas propellant enters into the valve housing by means of sufficient clearance between the walls of the valve body and the valve housing. The active ingredient and the gas propellant both enter into a mixing chamber through simultaneously openable and closable passage openings. The valve stem of the valve has two channels. One channel in the valve stem provides a flow path for the gas propellant and for the active ingredient mixture, the other channel in the stem providing a flow path substantially for the gas propellant alone. These channels combine in a second mixing chamber resulting in still further atomization of the spray and more efficient utilization of substantially the entire container content. US Patent 3 596 811 teaches a valve for use in connection with an aerosol spray container which holds in its bottom portion a mixture of liquid-gas propellants, an active ingredient and a solvent. the gas propellant is situated above these elements in the remaining free space of the container.

US Patent 4 463 905 entitled Foam-Generating Pump Sprayer, issued to Paul R. Stoesser, John E. Cuxie and John W. McLaren on August 7, 1984, teaches a hand-held apparatus for spraying foam which includes a manually dispensing pump capable of receiving a foamable liquid from a suitable container and ejecting it into the atmosphere through an atomizing nozzle.

U.S. Patent No. 4,407,842, entitled Method and Composition Rapidly Developing Latent Fingerprints, issued to Billy H. Shepart on October 4, 1985, teaches a method and composition for developing latent fingerprints on various surfaces including but not limited to glass, plastic, paper and various metals such as copper and brass. A mixture of cyanoacrylate ester, sodium bicarbonate and sulfur produces gaseous fumes to which the latent fingerprints are subjected. Cyanoacrylate ester is a commercially available adhesive sold under either the trademark "Super Glue" or the trademark "Eastman 910". In a very short period of time the gaseous fumes will develop the latent fingerprints so that the fingerprints can be easily lifted by conventional techniques for comparison with known fingerprints. The gaseous fumes are pumped into a portable container under pressure and are stored in the portable, pressurized container which includes a manually operable valve and nozzle arrangement in order to direct the gaseous fumes onto the surfaces which the latent fingerprints may be found.

U.S. Patent No. 4,297,383, entitled Apparatus and Method for Obtaining Fingerprints, issued to Louis P. Bourdon on October 27, 1981, teaches an apparatus and method for developing latent fingerprints on an object. The apparatus includes a first chamber which contains the object and which closes in order to seal the first chamber airtight and form a vapor tank and a second chamber which contains a chemical pool and vapors thereof. The apparatus also includes a pump system which pumps vapors from the second chamber into the vapor tank. The method includes the step of pumping the vapors into the vapor tank in order to fume the object with the vapors of the chemical cyanoacrylate and to develop the latent fingerprints on the object being tested inside the vapor tank. U.S. Patent No. 3,546,003 teaches a similar apparatus and method for obtaining latent fingerprints.

U.S. Patent No. 4,461,235, entitled Vapor Phase Activator Pad for a Self-Contained Fingerprinting Kit, issued to William P. Morton on July 24, 1984, teaches a vapor phase activator pad for use in a self-contained fingerprinting kit which includes a portable vapor tank into which the vapor phase activator pad is placed in order to fume an object suspected of containing latent fingerprints. The vapor phase activator pad includes a gauze pad which is chemically treated with a composition which contains a specified chlorinated organic solvent and a special organic amine so that when a quantity of the organic amine is placed onto the vapor phase activator pad, the vapor phase activator pad generates vapors of the chemical cyanoacrylate. The composition consists of a first component which is a mixture of two selective chemicals, trichloroethane in the range of 1 to 96 percent and stabilized with methanol in the range of 1 to 10 percent. The composition also includes a second component which is a mixture of four selective chemicals, nitroethane and nitromethane in the range of 1 to 5 percent and stabilized with toluene in the range of 1 to 4 percent, the second component also includes an organic amine, such as diethylenetriamine, which functions as an activator in the range of 1 to 9 percent wherein the composition provides a controlled oxidative vaporization of the quantity of alkylcyanoacrylate.

U.S. Patent No. 4,260,645, entitled Latent Fingerprint Detection, issued to F. Michael Kerr and Alan D. Westland on April 7, 1981, teaches a method of detecting and visualizing a latent fingerprint which includes the step of applying a solution to a suspected locale. The solution includes a volatile organic solvent and selected salts soluble in the volatile organic solvent. The salts include silver perchlorate and silver trifluoroacetate. The non-aqueous solution is preferably applied as a spray and minimizes smudging, "running", warping, and other damage to water-sensitive materials such as inks, dyes and/or cellulosic substrates.

U.S. Patent No. 4,258,073, entitled Taking of Fingerprints, issued to John M. Payne on March 24, 1981, teaches a method of revealing a fingerprint which includes the steps of charging a surface bearing the fingerprint to a high electric potential and applying a finely divided carbon to the charged surface in order to form a pattern thereon which corresponds to the fingerprint. The method of revealing a fingerprint also includes the steps of either dusting or spraying the finely divided carbon onto the charged surface and applying a transparent protective layer in order to fix the pattern of finely divided carbon in position. Alternatively the finely divided carbon may be in suspension in a dielectric liquid into which the charged surface is introduced.

U.S. Patent No. 4,176,205 entitled Fingerprint Powder and Method of Application, issued to Orlando G. Molina on November 27, 1979, teaches a fingerprint powder which includes a powder carrier which is a mixture of silica and talc and a coloring agent which is a fluorescent dye. The fingerprint powder is used to develop latent fingerprints.

U.S. Patent No. 2,066,535 entitled Fingerprint Detection, issued to Francis F. Lucas on January 5, 1937, also teaches the use of fluorescent dye for use in developing fingerprints.

U.S. Patent No. 4,381,159, entitled Magnetic Fingerprint Dusting Brush, issued to John M. Payne on April 26, 1983, teaches a magnetic fingerprint dusting brush which includes a handle which incorporates a magnetic portion that projects at one end thereof and a non-magnetic shroud which is assembled with the handle closely to shroud the projecting magnetic portion. The shroud includes an inner blind sleeve for closely shrouding the projecting magnetic portion and an outer sleeve to which a cover is detachably secured. The inner blind sleeve has a first portion of greater cross-section for assembly with the handle and a coaxial second portion of lesser cross-section which is connected to the first portion through a shoulder for closely shrouding the projecting magnetic portion. The magnetic fingerprint dusting brush also includes a cover which is detachably securable to the handle and shroud assembly to form in its secured position an enclosed powder reservoir around the shrouded magnetic portion of the handle. The shroud and the cover assembly constitute a powder cartridge with the handle. The powder reservoir contains a mixture of ferrous and dusting powders.

U.S. Patent No. 4,379,178, entitled Fingerprinting System, issued to Louis B. Meadows and Arthur S. Diamond on April 5, 1983, teaches a method for forming fingerprint images which includes the steps of prewetting and cleaning the portion of a finger with a cloth impregnated with a detergent solution and applying the distal portion of a finger to a porous pad impregnated with a solution of marking compound. The method for forming fingerprint images also includes the steps of applying the distal portion of the finger to a square of a fingerprint card impregnated with an aqueous solution of a polyhydroxy developer, such as a solution of 8-hydroxy quinoline and propyl gallate containing a high molecular weight dibasic acid, such as azelaic acid and, when the fingerprint image immediately develops, removing traces of the images with a cloth impregnated with a cleaning solution.

U.S. Patent No. 4,258,644, entitled Depositing Latent Fingerprints and Development Thereof, issued to Edward J. Goettert and George V. D. Tiers on March 31, 1981, teaches a method of depositing and developing a latent fingerprint which includes the steps of making a composition which includes a trimeric aliphatic acid of at least 30 carbon atoms which is substantive to paper fibers, substantially non-volatile, non-hardening, non-toxic and non-hygroscopic and using the composition to apply the fingerprint, which has a latency of several weeks, to a paper substrate. The method of depositing and developing a latent fingerprint also includes the steps of dusting the latent fingerprints in a conventional manner with suitable toner particles, such as magnetic particles in an oleophilic matrix, and developing the latent fingerprint.

U.S. Patent No. 3,523,628, entitled Container for Cyanoacrylate Adhesive, issued to Roger D. Colvin, John B. Duffy and Ross G. Wittemann on January 18, 1968, teaches a container for cyanoacrylate ester adhesive composition which include a container body and a dispensing nozzle. The container body is constructed from an air-impermeable material, preferably a metal such as aluminum. The dispensing nozzle has at least the dispensing surfaces thereof formed of a thermoplastic resin having low surface free energy. The container is designed to minimize deterioration of contents and afford long shelf stability.

U.S. Patent No. 4,103,081, entitled Stabilized Anaerobic Adhesives, issued to Stephen Repetto on July 25, 1978, teaches a curable anaerobic adhesive composition in which a chlorinated nitrobenzene stabilizer is incorporated as a stabilizer. U.S. Patent No. 4,295,909, entitled Curable Polybutadiene-Based Resins Having Improved Properties, issued to Louis J. Baccei on October 20, 1981, teaches a polymerizable adhesive and sealant composition.

U.S. Patent No. 3,678,015, entitled Low Temperature Peroxide Curable Thermosetting Polyurethane Resin Having Terminal Unsaturation, issued to Hisao Suzuki, Akira Musashi, Makoto Hiruta and Hiroo Muramoto on July 18, 1972, teaches a peroxide-curable thermosetting resin which includes an isocyanate compound and acrylate esters.

U.S. Patent No. 4,375,275, entitled High Viscosity Product Dispenser, issued to Dennis J. Argazzi on March 1, 1983, teaches a system for dispensing a highly viscous, adhesive material in metered amounts and without clogging which includes a body having a material discharge orifice and a discharge seat, a hollow valve spindle engageable with the discharge seat for shutting off the flow of the highly viscous, adhesive material through the material discharge orifice.

In view of the foregoing factors and conditions which are characteristic of the prior art, it is one object of the present invention to provide an aerosol spray container for use in a system for spraying a mist of atomized particles of cyanoacrylate ester in order to fume an object suspected of containing latent fingerprints thereon with vapors from the atomized particles of cyanoacrylate ester.

It is another object of the present invention to provide an aerosol spray container which is filled with a mixture of a chlorinated organic solvent, a cyanoacrylate ester and hydrocarbon propellant which the aerosol spray container discharges as a fine mist of atomized particles of the cyanoacrylate ester which are activated in order to generate rapid and prolonged fuming of the suspected object with the vapors of the atomized particles of cyanoacrylate ester.

It is still another object of the present invention to use a spray container with a manually operated-dispensing mechanism for use in a system for spraying a mist of atomized particles of cyanoacrylate ester in order to fume an object suspected of containing latent fingerprints thereon with vapors from the atomized particles of cyanoacrylate ester.

Another object involves the use of a portable system for rapidly, simply, and inexpensively identifying a person who wishes to make a credit purchase by means of an identity card, such as credit card or bank card.

It is yet another object of the present invention to use a spray container with either a manually operated-dispensing mechanism or an aerosol dispensing mechanism for spraying a mist of atomized particles of cyanoacrylate ester so that it is not necessary to use the spray container in a vapor tank in order to fume an object with vapors from the atomized particles of cyanoacrylate ester.

The spray container of the present invention is characterized in that a mixture of said cyanoacrylate ester and a chlorinated organic solvent is dispersed in said hydrocarbon propellant, said chlorinated organic solvent evaporating from said atomizate to release vapors of said cyanoacrylate therefrom.

In accordance with one preferred embodiment of the present invention a spray container for spraying a mist of atomized particles of cyanoacrylate ester for use in a self-contained fingerprinting kit is described. The spray container includes a container, a dispensing mechanism which is coupled to the container, and a valve with a nozzle. A liquid cyanoacrylate ester is disposed in the container. The liquid cyanoacrylate is discharged as a mist of atomized particles through the nozzle of the valve of the container. The spray container discharges the mist of the particles of cyanoacrylate ester in order to generate vapors of the chemical cyanoacrylate thereby fuming an object suspected of containing latent fingerprints.

It is yet another object of the present invention to provide a spray container for applying a cyanoacrylate ester adhesive composition onto two opposing surfaces which are chemically joined together wherein the spray container is filled with the cyanoacrylate ester adhesive composition which the spray container atomizes into particles and discharges as a fine mist of the particles onto the two opposing surfaces.

It is still another object of the present invention to use a spray container with a manually operated-dispensing mechanism for use in a system for spraying a mist of atomized particles of a cyanoacrylate ester adhesive composition.

In accordance with another preferred embodiment of the present invention a spray container for use in applying a cyanoacrylate ester adhesive composition which is a liquid and which is disposed in the spray container is described. The spray container includes a container which is constructed from an air-impermeable material and which has a valve with a nozzle. The spray container also includes a spraying mechanism which has a spray head with one or more apertures or orifices and a cylinder with a piston. The movement of the piston in the cylinder draws the liquid into the spray head in order to atomize and spray the liquid through the apertures upon forward movement of the piston. The spray container discharges the fine mist of atomized particles of the cyanoacrylate ester adhesive composition onto at least one of the two opposing surfaces which are to be chemically joined together.

In accordance with the present invention a yet further embodiment of a self-contained fingerprinting system which includes a spray container and a portable vapor tank is described. The spray container discharges into the portable vapor container a mist of atomized particles of cyanoacrylate ester in order to generate vapors thereof. The vapors in the portable vapor tank fume an object suspected of containing latent fingerprints. The portable vapor tank includes a shroud which encloses the vapors from the mist of atomized particles of cyanoacrylate ester, a first supporting member which supports the shroud and a second supporting member which supports the object in front of the spray container. The portable vapor tank also includes a timer which determines the duration of fuming with the vapors and purging apparatus which purges the vapors from the portable vapor tank. The purging apparatus is electrically coupled to the timer and activated thereby at the end of duration of fuming with the vapors. When a fluorescent dye is added to the mist of atomized particles of cyanoacrylate ester in order to generate fluorescent vapors, the portable vapor tank further includes an ultraviolet source which exposes latent fingerprints when the ultraviolet light source is shined on the object which contains the latent fingerprints and which has been treated with the fluorescent vapors.

Other claims and many of the attendant advantages will be more readily appreciated as the same becomes better understood by reference to the following detailed description and considered in connection with the accompanying drawing in which like reference symbols designate like parts throughout the figures.

Fig. 1 is a side elevational view in cross-section of an aerosol spray container which is for use in a system for fuming an object suspected of containing latent fingerprints thereon with vapors generated from atomized particles of cyanoacrylate ester and which also can be used in applying a cyanoacrylate ester adhesive composition onto at least one of two opposing surfaces which are to be chemically joined together and which has been constructed in accordance with the principles of the present invention.

Fig. 2 is a perspective drawing of the aerosol spray container of Fig. 1 showing it discharging into a portable vapor tank a fine mist of a mixture of a chlorinated organic solvent and a cyanoacrylate ester along with the hydrocarbon propellant in order to generate vapors from the particles of cyanoacrylate ester in order to fume an object suspected of containing latent fingerprints.

Fig. 3 is a perspective drawing of a fingerprinting system which has been constructed in accordance with the principles of the present invention and which includes a portable vapor tank and a spray container which is used for fuming an object suspected of containing latent fingerprints thereon with vapors generated from atomized particles of cyanoacrylate ester in the portable vapor tank.

Fig. 4 is a top view of the fingerprinting system of Fig. 3 in which the portable vapor tank is open in order to expose the internal components of the fingerprinting system.

Fig. 5 is a front elevational view of the fingerprinting system of Fig. 3.

Fig. 6 is a side elevational view in cross-section of the fingerprinting system of Fig. 3 taken along the line 6—6 of Fig. 5.

Fig. 7 is a side elevational view in cross-section of the fingerprinting system of Fig. 3 taken along line 7—7 of Fig. 5.

Fig. 8 is a front elevational view in cross-section of the fingerprinting system of Fig. 3.

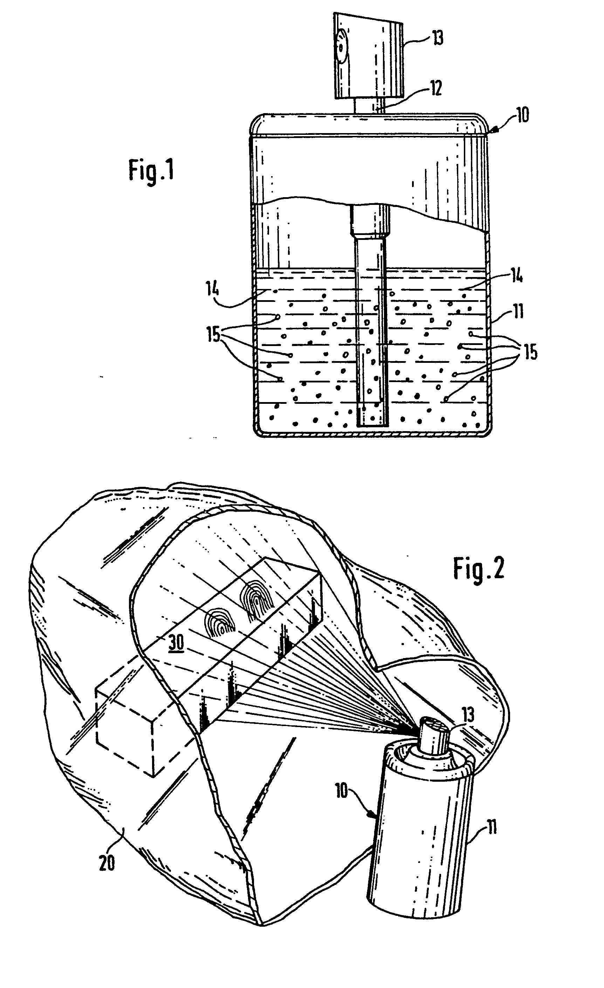

In order to best understand the present invention it is necessary to refer to the following description of its preferred embodiment. Referring to Fig. 1 aerosol spray container 10 includes container 11 which has valve 12 with nozzle 13. Hydrocarbon propellant 14 is disposed in container 11. Mixture 15 of a chlorinated organic solvent and a cyanoacrylate ester chemical is disposed in the container 11. Mixture 15 is discharged along with hydrocarbon propellant 14 through nozzle 13 of valve 12 of container 11.

Referring to Fig. 2 in conjunction with Fig. 1 aerosol spray container 10 is for use in a self-contained fingerprinting kit which includes portable vapor tank 20. Aerosol spray container 10 discharges into portable vapor tank 20 a fine mist of the mixture 15 of the chlorinated organic solvent and the cyanoacrylate ester along with hydrocarbon propellant 14. The fine mist of mixture 15 of the chlorinated organic solvent and the cyanoacrylate ester generates vapors from the atomized particles of cyanoacrylate ester thereby fuming object 30 suspected of containing latent fingerprints.

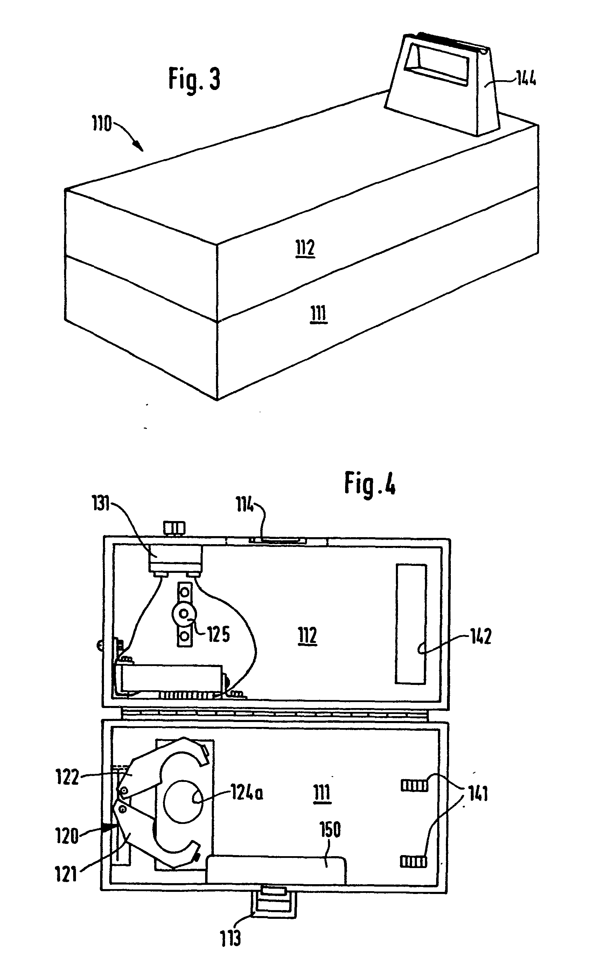

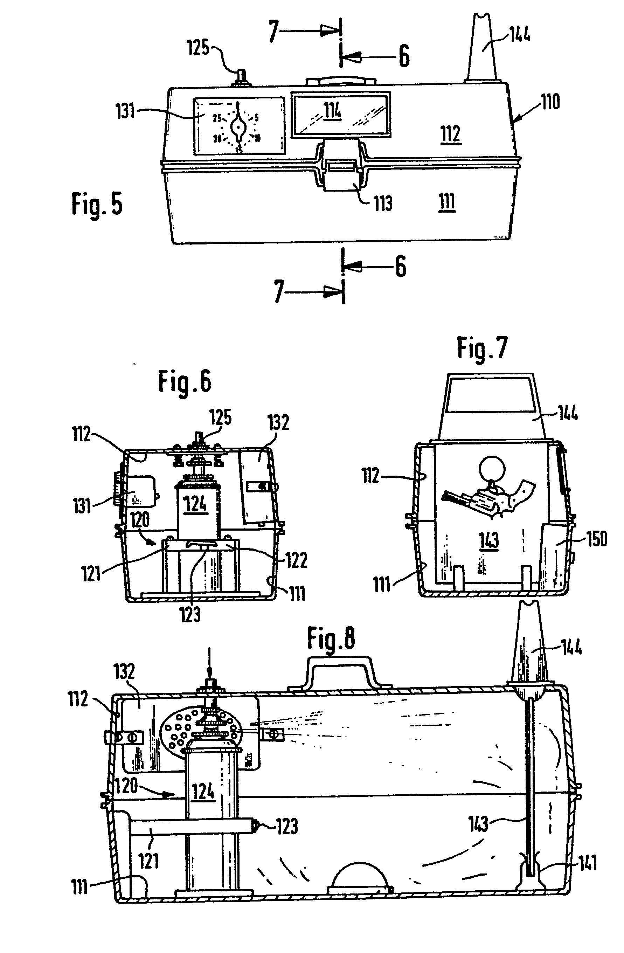

As an alternative to the embodiment in Fig. 2, and referring to Fig. 3 in conjunction with Fig. 4 and Fig. 5, a self-contained fingerprinting system 110 includes a portable vapor tank having a base 111 and a top 112 which is hinged to the base 111. In their close position the base 111 and the top 112 are also mechanically coupled by a first latch 113. The top 112 has a viewing window 114.

Referring to Fig. 6 in conjunction with Fig. 4 and Fig. 5, a self-contained fingerprinting system 110 also includes a container holder 120 which has a first arm 121 and a second arm 122 both of which are mechanically hinged to the base 111. A second latch 123 mechanically secures the first and second arms 121 and 122 together. A spray container 124 is placed in a container-holding hole 124a. A push-button is disposed in the top 112 so that it is able to trigger the spray container 124. The spray container 124 discharges into the portable vapor tank 110 a mist of atomized particles of cyanoacrylate ester in order to generate vapors thereof. The vapors in the portable vapor tank fume an object suspected of containing latent fingerprints. The portable vapor tank 110 may include a shroud which is formed out of plastic and which effectively extends the length of the portable vapor tank 110 in order to enclose the vapors from the mist of atomized particles of cyanoacrylate ester. The portable vapor tank 110 also includes a timer 131 which determines the duration of fuming with the vapors and a purging apparatus 132 which purges the vapors from the portable vapor tank 110. The purging apparatus 132 is electrically coupled to the timer and activated thereby at the end of duration of fuming with the vapors. The purging apparatus 132 includes a vacuuming device which has an inlet and an outlet which is fluidly coupled to the inside of the portable vapor tank 110 and a charcoal filter which is fluidly coupled to the inlet of the vacuuming device in order to collect the vapors from the inside of the portable vapor tank 110.

Referring to Fig. 7 in conjunction with Fig. 3, Fig. 4, Fig. 5, and Fig. 8, a self-contained fingerprinting system 110 further includes a supporting apparatus which supports the object in front of the spray container 124. The supporting apparatus includes a pair of stops 141 which are disposed on the base 111 and a rectangular hole in the top which is aligned with the pair of stops 141. The supporting apparatus also includes a slide plate 143 and a stopper 144 which is mechanically coupled to the slide plate 143 and which is to be inserted into the rectangular hole 142 in the top 112.

Referring to Fig. 4 in conjunction with Fig. 5 and Fig. 6, a self-contained fingerprinting system 110 when a fluorescent dye is added to the mist of atomized particles of cyanoacrylate ester in order to generate fluorescent vapors the portable vapor tank 110 further includes an ultraviolet source 150 which exposes latent fingerprints when the ultraviolet light source 150 is shined on the object which contains the latent fingerprints and which has been treated with the fluorescent vapors.

The organic solvent when mixed with the cyanoacrylate ester reduces it to atomized particles which the hydrocarbon propellant can easily transport.

A fluorescent dye, such as Rhodamine B, may be added to mixture 15 of the chlorinated organic solvent and the alkyl-cyanoacrylate chemical in order to generate fluorescent vapors of the atomized particles of cyanoacrylate ester. Another fluorescent dye, such as Hostasol yellow 8G, which American Hoechst Corporation manufactures, may also be used. An ultraviolet source exposes latent fingerprints when the ultraviolet light source is shined on the object containing the latent fingerprints.

U.S. Patent No. 4,227,631 teaches a valve for an aerosol spray container which has a mixing chamber for mixing a gas propellant with an active ingredient in order to produce a finely atomized spray. U.S. Patent No. 3,596,811 teaches a valve for use in connection with an aerosol spray container which holds in its bottom portion a mixture of liquid-gas propellants, an active ingredient and a solvent.

In another embodiment the spray container may include a container which has a valve with a nozzle. A mixture of a chlorinated organic solvent and a cyanoacrylate ester is disposed in the container. A discharging mechanism discharges a fine mist of atomized particles of the mixture of the chlorinated organic solvent and the cyanoacrylate ester through the nozzle. The vapors generated from the atomized particles of the cyanoacrylate ester can be used to fume an object suspected of containing latent fingerprints. A fluorescent dye is added to the mixture of the chlorinated organic solvent and the cyanoacrylate ester in order to generate fluorescent vapor fumes so that the latent fingerprints become exposed when an ultraviolet light source is shined on the object containing the latent fingerprints.

U.S. Patent No. 4,243,159 teaches a spring actuated pump device including a discharge valve for effecting spraying of a desired quantity of fluid for the spring actuated pump device. U.S. Patent No. 4,463,905 teaches a hand-held apparatus for spraying foam through an atomizing nozzle.

U.S. Patent No. 3,056,560 entitled Liquid Sprayer, issued to William Martin Vogel, Jr. on October 2, 1962, teaches a liquid sprayer which includes a spray head with one or more apertures or orifices and a cylinder with a piston. The movement of the piston in the cylinder draws the liquid into the spray head. The liquid sprayer atomizes and sprays a liquid through the apertures upon forward movement of the piston. The liquid sprayer is of a general type which U.S. Patent No. 2,194,339, U.S. Patent No. 2,233,161, and U.S. Patent No. 2,178,088 teach.

The discharging mechanism is manually operated and may be similar to those discharging mechanisms which U.S. Patent No. 4,243,159, U.S. Patent No. 4,463,905, and U.S. Patent No. 3,506,560 describe.

From the foregoing it can be seen that a spray container for use in a system for fuming an object suspected of containing latent fingerprints thereon with vapors generated from atomized particles of cyanoacrylate ester has been described.

Aerosol spray container 10 also may be used in applying an adhesive material. Aerosol spray container 10 discharges a fine mist of mixture 15 of the chlorinated organic solvent and cyanoacrylate ester chemical along with hydrocarbon propellant 14 onto at least one of two opposing surfaces which are chemically joined together. In this alternative embodiment of the present invention, the spray container is used for application of a cyanoacrylate ester adhesive composition which is a liquid and which is disposed in the spray container. The cyanoacrylate ester adhesive composition is treated with an organic acid including a fatty acid, such as a stearic acid, oleic acid, palmitic acid, lauric acid, or other acid such as maleic acid, phthalic acid, and oxalic acid; or with an inorganic acid, such as phosphoric acid, nitric acid, hydrochloric acid, sulfuric acid, or fluorophosphoric acid, provided that the pH is in the range of 1.5 to 6.8 and preferably in the range of 3.0 to 6.5. The spray container includes a container which is constructed from an air-impermeable material and which has a valve with a nozzle. The air-impermeable material may include either plastic materials such as polypropylene, polyethylene, nylon, polyacetate, or teflon; or metallic materials, such as aluminum. The internal surfaces of the container and the valve are treated with a coating such as an oil, silicone grease, or hydrocarbon grease. The spray container also includes a spraying mechanism which has a spray head with one or more apertures or orifices and a cylinder with a piston. The movement of the piston in the cylinder draws the liquid into the spray head in order to atomize and spray the liquid through the apertures upon forward movement of the piston. The spray container discharges the fine mist of atomized particles of the cyanoacrylate ester adhesive composition onto at least one of two opposing surfaces which are to be chemically joined together. In still another embodiment the internal surfaces of the container and valve may be treated with one of these acids.

From the foregoing it can be seen that a spray container for use in applying a cyanoacrylate ester adhesive composition onto two opposing surfaces which are to be chemically joined together has been described.

Accordingly, it is intended that the foregoing disclosure and showing made in the drawings shall be considered only as an illustration of the principles of the present invention.

## Claims

1. A spray container (10, 124) for generating vapors of cyanoacrylate ester comprising a container (11) fitted with a discharge mechanism (12 and 13, 125) in which is disposed cyanoacrylate ester dispersed in a hydrocarbon propellant (14) wherein actuating the discharge mechanism (12 and 13, 125) generates an atomizate of said cyanoacrylate ester characterized in that a mixture (15) of said cyanoacrylate ester and a chlorinated organic solvent is dispersed in said hydrocarbon propellant (14), said chlorinated organic solvent evaporating from said atomizate to release vapors of said cyanoacrylate therefrom.

2. The spray container (10, 124) of claim 1 characterized in that said solvent is selected from the group consisting of dichloro fluoromethane, dichloro tetrafluoroethane, trichloro ethane, trichloro fluoromethane, and mixtures thereof.

3. The spray container (10, 124) of claim 1 characterized in that said mixture (15) additionally contains a fluorescent dye in order to generate fluorescent vapor fumes for fuming an object suspected of containing latent fingerprints whereby said latent fingerprints become visibly perceptible when an ultraviolet light source is shined on said object containing said latent fingerprints.

4. The spray container (10, 124) of claim 1 characterized in that said atomizate of said mixture (15) is directed onto at least one of two opposing surfaces which are to be joined together.

5. The spray container (124) of claim 1 characterized in that said container is disposed in a portable vapor tank fitted with a container holder (120) for holding said container, said portable vapor tank also comprising supporting means (141, 143, 144) for supporting an object in front of said spray container (124) and a push-button (125) disposed outside of said vapor tank so that it is able to trigger said container discharge mechanism (125) to discharge a mist of atomized particles in order to generate vapors of said cyanoacrylate ester for fuming an object which is suspected of containing latent fingerprints.

6. The spray container (124) of claim 5 characterized in that said vapor tank also comprises shrouding means for enclosing said vapors from said mist of atomized particles of cyanoacrylate ester in order to fume with said vapors an object suspected of containing latent fingerprints; and

first supporting means for supporting said shrouding means and said object in front of said discharging means (125) with said supporting means being mechanically coupled to the top of said portable vapor tank.

7. The spray container (124) of claim 5 characterized in that said portable vapor tank also comprises timing means (131) for determining the duration of fuming with said vapor; and

purging means (132) for purging said vapors from said portable vapor tank, said purging means (132) being electrically coupled to said timing means (131) and activated thereby at the end of duration of fuming with said vapors.

8. The spray container (124) of claim 7 characterized in that said purging means (132) comprises:

a vacuuming device which has an inlet and an outlet which is fluidly coupled to the inside of said portable vapor tank;

a charcoal filter which is fluidly coupled to said inlet of said vacuuming device in order to collect said vapors from the inside of said portable vapor tank.

9. The spray container (124) of claim 5 characterized in that said portable vapor tank also comprises an ultraviolet source which exposes latent fingerprints when the ultraviolet light source is shined on the object which contains the latent fingerprints and which has been treated with said fluorescent vapors.

## Patentansprüche

1. Sprühdose (10, 124) zum Erzeugen von Dämpfen von Cyanacrylatester, mit einer Dose (11), die mit einer Abgabevorrichtung (12 und 13, 125) versehen ist, in der der Cyanacrylatester dispergiert in einem Kohlenwasserstofftreibmittel (14) angeordnet ist, wobei das Betätigen der Abgabevorrichtung (12 und 13, 125) einen Nebel des Cyanacrylatesters erzeugt, dadurch gekennzeichnet, daß eine Mischung (15) des Cyanacrylatesters und eines chlorierten organischen Lösungsmittels in dem Kohlenwasserstofftreibmittel (14) dispergiert ist, wobei das chlorierte organische Lösungsmittel aus dem Nebel verdampft, um Dämpfe des Cyanacrylats daraus freizusetzen.

2. Sprühdose (10, 124) nach Anspruch 1, dadurch gekennzeichnet, daß das Lösungsmittel aus der Gruppe ausgewählt ist, die aus Dichlorfluormethan, Dichlortetrafluorethan, Trichlorethan, Trichlorfluormethan und Gemischen derselben besteht.

3. Sprühdose (10, 124) nach Anspruch 1, dadurch gekennzeichnet, daß die Mischung (15) zusätzlich einen Fluoreszenzfarbstoff enthält, um fluoreszierende Dämpfe zum Einnebeln eines Objekts zu erzeugen, das verdächtigt wird, verborgene Fingerabdrücke aufzuweisen, wodurch die verborgenen Fingerabdrücke sichtbar werden, wenn eine UV-Lichtquelle das Objekt bestrahlt, das die verborgenen Fingerabdrücke aufweist.

4. Sprühdose (10, 124) nach Anspruch 1, dadurch gekennzeichnet, daß der Nebel der Mischung (15) auf wenigstens eine von zwei einander gegenüberliegenden Oberflächen gerichtet wird, die miteinander verbunden werden sollen.

5. Sprühdose (124) nach Anspruch 1, dadurch gekennzeichnet, daß die Dose in einem tragbaren Dampfbehälter angeordnet ist, der mit einem Dosenhalter (120) zum Festhalten der Dose versehen ist, wobei der tragbare Dampfbehälter außerdem eine Trageinrichtung (141, 143, 144) aufweist, um ein Objekt vor der Sprühdose (124) zu halten, und einen Druckknopf (125), der außerhalb des Dampfbehälters angeordnet ist, so daß er in der Lage ist, die Dosenabgabevorrichtung (125) zu betätigen, um einen Nebel von zerstäubten Partikeln abzugeben, um Dämpfe des Cyanacrylatesters zum Einnebeln eines Objekts zu erzeugen, welches verdächtigt wird, verborgene Fingerabdrücke aufzuweisen.

6. Sprühdose (124) nach Anspruch 5, dadurch gekennzeichnet, daß der Dampfbehälter außerdem eine Umhüllungseinrichtung aufweist zum Einschließen der Dämpfe aus dem Nebel von zerstäubten Partikeln des Cyanacrylatesters, um mit den Dämpfen ein Objekt einzunebeln, das verdächtigt wird, verborgene Fingerabdrücke aufzuweisen; und

eine erste Trageinrichtung zum Halten der Umhüllungseinrichtung und des Objekts vor der Abgabeeinrichtung (125), wobei die Trageinrichtung mit dem Oberteil des tragbaren Dampfbehälters mechanisch verbunden ist.

7. Sprühdose (124) nach Anspruch 5, dadurch gekennzeichnet, daß der tragbare Dampfbehälter außerdem eine Zeitsteuereinrichtung (131) aufweist zum Festlegen der Dauer des Einnebelns mit dem Dampf; und

eine Reinigungseinrichtung (132) zum Austreiben der Dämpfe aus dem tragbaren Dampfbehälter, wobei die Reinigungseinrichtung (132) mit der Zeitsteuereinrichtung (131) elektrisch verbunden ist und durch diese am Ende der Zeitspanne des Einnebelns mit den Dämpfen aktiviert wird.

8. Sprühdose (124) nach Anspruch 7, dadurch gekennzeichnet, daß die Reinigungseinrichtung (132) aufweist:

eine Vakuumvorrichtung, die einen Einlaß und einen Auslaß hat, der mit dem Inneren des tragbaren Dampfbehälters in Fluidverbindung ist;

einen Aktivkohlefilter, der mit dem Einlaß der Vakuumvorrichtung in Fluidverbindung steht, um die Dämpfe aus dem Inneren des tragbaren Dampfbehälters zu sammeln.

9. Sprühdose (124) nach Anspruch 5, dadurch gekennzeichnet, daß der tragbare Dampfbehälter außerdem eine Ultraviolett-Quelle aufweist, welche verborgene Fingerabdrücke sichtbar macht, wenn die Ultraviolettquelle auf das Objekt gerichtet wird, welches die verborgenen Fingerabdrücke aufweist und mit den Fluoreszenzdämpfen behandelt worden ist.

**Revendications**

1. Récipient de pulvérisation (10, 124) pour la production de vapeurs d'ester cyano-acrylate, comprenant un récipient muni d'un mécanisme d'évacuation (12 et 13, 125) dans lequel est disposé l'ester cyano-acrylate mis en dispersion dans un agent propulseur (14) hydrocarboné, la mise en oeuvre du mécanisme d'évacuation (12 et 13, 125) produisant un atomisat de cet ester cyano-acrylate, caractérisé en ce que l'on met un mélange (15) constitué de cet ester cyano-acrylate et d'un solvant organique chloré en dispersion dans l'agent propulseur (14) hydrocarboné, le solvant organique chloré s'évaporant de cet atomisat pour en libérer des vapeurs de cyano-acrylate.

2. Récipient de pulvérisation (10, 124) selon la revendication 1, caractérisé en ce que le solvant est choisi parmi le groupe comprenant le dichlorofluorométhane, le dichlorotétrafluoréthane, le trichloréthane, le trichlorofluorométhane et des mélanges de ces derniers.

3. Récipient de pulvérisation (10, 124) selon la revendication 1, caractérisé en ce que le mélange (15) contient, en outre, un colorant fluorescent en vue de produire des vapeurs fluorescentes auxquelles on expose un objet susceptible de contenir des empreintes digitales latentes, ces empreintes digitales latentes devenant perceptibles à la vue lorsqu'une source de lumière ultraviolette éclaire l'objet contenant les empreintes digitales latentes.

4. Récipient de pulvérisation (10, 124) selon la revendication 1, caractérisé en ce que l'on dirige l'atomisat constitué du mélange (15) sur au moins une de deux surfaces opposées devant être réunies l'une à l'autre.

5. Récipient de pulvérisation (124) selon la revendication 1, caractérisé en ce que le récipient est disposé dans un réservoir de vapeurs portatif muni d'un organe (120) destiné à maintenir le récipient, le réservoir de vapeurs portatif comprenant également des moyens (141, 143, 144) pour supporter un objet devant le récipient de pulvérisation (124) et un bouton poussoir (125) disposé à l'extérieur du réservoir de vapeurs, de telle sorte qu'il puisse déclencher le mécanisme (125) d'évacuation du récipient, en vue d'évacuer un brouillard de particules atomisées pour produire des vapeurs d'ester cyano-acrylate auxquelles on expose un objet suspecté de contenir des empreintes digitales latentes.

6. Récipient de pulvérisation (124) selon la revendication 5, caractérisé en ce que le réservoir de vapeurs comprend également un moyen d'enveloppe pour enfermer les vapeurs constituées du brouillard de particules atomisées d'ester cyano-acrylate afin d'exposer à ces vapeurs, un objet suspecté de contenir des empreintes digitales latentes; et

un premier moyen pour supporter ce moyen d'enveloppe et cet objet devant le moyen d'évacuation (125), le moyen de support étant mécaniquement couplé au sommet du réservoir de vapeurs portatif.

7. Récipient de pulvérisation (124) selon la revendication 5, caractérisé en ce que le réservoir de vapeurs portatif comprend également un moyen de minuterie (131) destiné à déterminer la durée de l'exposition à ces vapeurs; et

un moyen de purge (132) pour chasser les vapeurs hors du réservoir de vapeurs portatif, ce moyen de purge (132) étant électriquement couplé au moyen de minuterie (131) et activé ainsi au terme de l'exposition aux vapeurs.

8. Récipient de pulvérisation (124) selon la revendication 7, caractérisé en ce que le moyen de purge (132) comprend:

un dispositif d'aspiration muni d'une entrée et d'une sortie, celle-ci étant couplée en écoulement à l'intérieur du réservoir de vapeurs portatif;

un filtre en charbon de bois couplé en écoulement à l'entrée du dispositif d'aspiration en vue de recueillir les vapeurs provenant de l'intérieur du réservoir de vapeurs portatif.

9. Récipient de pulvérisation (124) selon la revendication 5, caractérisé en ce que le réservoir de vapeurs portatif comprend également une source d'ultraviolet, à laquelle on expose les empreintes digitales latentes lorsque la source de lumière ultraviolette éclaire l'objet contenant les empreintes digitales, celles-ci ayant été traitées avec les vapeurs fluorescentes.

Fig.1

Fig.2

Fig. 3

110

144

112

111

Fig. 4

131

114

125

112

142

122

124a

120

121

111

150

141

113

Fig. 5

Fig. 6

Fig. 7

Fig. 8